# EUROPEAN PATENT APPLICATION

(11) **EP 4 302 758 A1**
(43) Date of publication of application: **10.01.2024**
(21) Application number: 23183430.0
(22) Date of filing: 04.07.2023
(51) Int. Cl.: A61K 31/185, A61K 45/06, A61K 33/06, A61K 31/195, A61K 33/14, A61K 31/7016, A61P 1/12, A23L 33/175

(54) **ORAL REHYDRATION COMPOSITION COMPRISING LACTOSE, SODIUM CHLORIDE, SODIUM BICARBONATE, GLYCINE, POTASSIUM AND ZEOLITE AND ITS USE IN TREATING GASTROINTESTINAL DISORDERS**

(30) Priority: 04.07.2022 IT 202200014092
(71) Applicant: Farmacia del Corso Snc di Ferri Sonia & C., 41013 Castelfranco Emilia Modena (IT)
(72) Inventor: FERRI, Sonia, 41013 Castelfranco Emilia (Modena) (IT); FERRI, Fabiana, 40053 Valsamoggia (Bologna) (IT)
(74) Representative: Delbarba, Andrea

(57) **Abstract**

The present invention relates to a composition comprising a disaccharide, a salt of hydrochloric acid, a salt of carbonic acid, at least one amino acid with a hydrophobic side chain, an alkali metal and an aluminosilicate. Furthermore, the invention relates to the use of the composition per the treatment or prevention of a gastrointestinal disorder or condition caused by a gastrointestinal disorder in an individual, preferably an animal.

## Description

### FIELD OF THE INVENTION

The present invention relates to a composition comprising a disaccharide, a salt of hydrochloric acid, a salt of carbonic acid, at least one amino acid with a hydrophobic side chain, an alkali metal and an aluminosilicate.

### STATE OF THE ART

Diarrhoea is a very common disorder in calves in the first weeks of life. All over the world, most of the mortality of calves is caused by this disorder. A recent study conducted by the American "National Animal Health Monitoring System (NAHMS)" showed that 23.9 percent of calves are affected at least once by diarrhoea. Even more surprising is that diarrhoea is the cause of 56.5 percent of deaths in the period of life between birth and weaning.

Considering these data, one is naturally led to think that on cattle farms it is necessary to implement all the management interventions whose objective is to reduce the prevalence of this disorder with the aim of limiting the huge economic losses.

Adequate prevention, early diagnosis and an equally early therapeutic intervention are important in order to limit the negative effects of this disorder.

The most common symptoms of diarrhoea include watery faeces and possibly dehydration.

When the diarrhoea becomes more serious, or persists for a long time, calves generally start to manifest: rough skin, dry nose and sunken eyes. As the dehydration worsens, the symptoms may be intensified, and the calf may also manifest anorexia and hypothermia. Finally, the animal may have difficulty in reaching and maintaining a standing position on all four legs. In the most serious cases, the calf manifests permanent recumbency and loss of conscience.

The progression of the disease and thus the worsening of symptoms are often very rapid; also for this reason, alongside prevention, prompt intervention is very important in the management of calves. In particular, there is a greatly felt need for a therapy that can be administered to the animal and allows it to rapidly rehydrate and recover from the electrolyte imbalance.

### SUMMARY OF THE INVENTION

A first aspect of the present invention relates to a composition that comprises or consists of: a disaccharide, a salt of hydrochloric acid, a salt of carbonic acid, at least one amino acid, preferably with a hydrophobic side chain, an alkali metal and an aluminosilicate.

A **second** aspect of the present invention relates to the composition as described above for use as a medicament.

A **third** aspect of the present invention relates to the composition as described above for use in the treatment or prevention of a gastrointestinal disorder or condition caused by a gastrointestinal disorder in an individual. A **fourth** aspect of the present invention relates to a meal replacement comprising the composition as described above. Preferably, the meal replacement is a replacement for a milk meal of an animal, preferably a calf.

### DETAILED DESCRIPTION OF THE INVENTION

A first aspect of the present invention relates to a composition that comprises or consists of a disaccharide, a salt of hydrochloric acid, a salt of carbonic acid, at least one amino acid preferably with a hydrophobic side chain, an alkali metal and an aluminosilicate.

Preferably, the disaccharide is present in a concentration of between 35% and 60% weight/weight (w/w), more preferably between 40% and 55% w/w.

Preferably, the salt of hydrochloric acid is present in a concentration of between 3.5% and 6% w/w, more preferably in a concentration of between 4% and 5.5% w/w.

Preferably, the salt of carbonic acid is present in a concentration of between 8% and 20% w/w, more preferably in a concentration of between 10% and 15% w/w.

Preferably, the at least one amino acid is present in a concentration of between 3.5% and 6% w/w, more preferably in a concentration of between 4% and 5% w/w.

Preferably, the alkali metal is present in a concentration of between 1% and 6% w/w, more preferably in a concentration of between 1.5% and 4% w/w.

Preferably, the aluminosilicate is present in a concentration of between 13% and 30%, more preferably in a concentration of between 15% and 25%.

In one embodiment, the composition further comprises at least one preservative of natural origin. Preferably, the at least one preservative of natural origin is present in a concentration of between 5 and 12% w/w, preferably between 7 and 10% w/w.

In one embodiment, the at least one preservative is citric acid.

In one embodiment, the disaccharide is selected from: lactose, sucrose, maltose, trehalose and combinations thereof. The disaccharide is preferably lactose.

In one embodiment, the salt of hydrochloric acid is sodium chloride and the salt of carbonic acid is sodium bicarbonate.

Preferably, the at least one amino acid is an amino acid with a hydrophobic side chain, preferably selected from: glycine, alanine, valine, isoleucine, leucine and methionine. The at least one amino acid with a hydrophobic side chain is preferably glycine.

In one embodiment, the alkali metal is selected from sodium and potassium; it is preferably potassium.

In one embodiment, the aluminosilicate is a zeolite.

In one embodiment of the invention, the composition is formulated for oral use, preferably in the form of a powder, preferably a granular powder to be reconstituted in a solvent, for example water, prior to use.

In an alternative embodiment, the composition is formulated as a liquid, preferably as a suspension or solution or as a syrup or beverage.

In a further embodiment, the composition is added to a food.

The Applicant has surprisingly found that the composition of the present invention is capable of hydrating or rehydrating an individual, preferably an animal, following a gastrointestinal condition or disorder. In particular, the Applicant has discovered that by administering the composition of the present invention to an individual affected by a gastrointestinal disorder, for example diarrhoea, and a consequent electrolyte imbalance, state of dehydration and metabolic acidosis, one obtains a rehydration of the individual. Furthermore, the present composition can be used as a meal replacement for an individual, preferably an animal affected by said gastrointestinal disorder. In addition, the composition of the present invention comprises only compounds of natural origin and, therefore, can also be used on organic farms.

A **second** aspect of the present invention relates to the composition as described in detail above for use as a medicament.

A **third** aspect of the present invention relates to the composition as described in detail above for use in the treatment or prevention of a gastrointestinal disorder or condition caused by a gastrointestinal disorder in an individual.

In one embodiment, the gastrointestinal disorder is selected from: diarrhoea, dysentery, inflammatory bowel disease (IBD), ulcerative colitis, intestinal colopathy and combinations thereof.

In one embodiment, the condition caused by a gastrointestinal disorder is selected from electrolyte dehydration, preferably dehydration following diarrhoea and/or dysentery and metabolic acidosis.

In one embodiment, the individual is an animal, preferably a dairy animal. Preferably, the animal is a bovine or a sheep or else a pig, more preferably a bovine. In one embodiment, the individual is a calf.

In one embodiment the composition is administered at least once a day, more preferably at least twice a day or as needed.

In a further embodiment, the composition is administered in association or in combination with a therapy for the treatment of the gastrointestinal disorder or condition caused by the gastrointestinal disorder. For example, the composition is administered in association or in combination with probiotics, prebiotics, or at least one drug to prevent or treat the gastrointestinal disorder.

A **fourth** aspect of the present invention relates to a meal replacement comprising the composition as described in detail above. Preferably, the meal replacement is a replacement for a milk meal of an animal, preferably a calf.

### EXAMPLE

### Preparation of the composition in powder form

The potassium chloride and sodium chloride were sieved in order to eliminate any lumps due to atmospheric humidity.

The components listed in Table 1 were added in a mixer, starting from the compounds in the smallest amounts, mixed for about 10 minutes in one direction and for another 10 minutes in the opposite direction.

**Table 1**

| **Compound** | **Weight (grams)** |
|---|---|
| Lactose | 486.00 |
| Sodium chloride | 48.00 |
| Sodio bicarbonate | 138.00 |
| Citric acid | 79.00 |
| Glycine | 46.00 |
| Potassium | 23.00 |
| Zeolite | 180.00 |

Once the composition was prepared, it was dissolved in water in order to be administered to the animal. Thanks to the citric acid, the solution becomes effervescent.

Fifty grams of powder as obtained above were dissolved in one litre of water. The solution obtained was used as a replacement for the daily milk meal of calves with diarrhoea and, therefore, consequent losses of electrolytes and states of dehydration and metabolic acidosis.

## Claims

1. A composition comprising a disaccharide, a salt of hydrochloric acid, a salt of carbonic acid, at least one amino acid preferably having a hydrophobic side chain, an alkali metal and an aluminosilicate wherein the disaccharide is present in a concentration between 35% and 60% w/w, the salt of hydrochloric acid is present in a concentration between 3.5% and 6% w/w, the salt of carbonic acid is present in a concentration between 8% and 20% w/w, the at least one amino acid is present in a concentration between 3.5% and 6% w/w, the alkali metal is present in a concentration between 1% and 6% w/w and the aluminosilicate is present in a concentration between 13% and 30%.

2. The composition according to claim 1, wherein the disaccharide is present in a concentration between 40% and 55% w/w, the salt of hydrochloric acid is present in a concentration between 4% and 5.5% w/w, the salt of carbonic acid is present in a concentration between 10% and 15% w/w, at least one amino acid is present in a concentration between 4% and 5% w/w, the alkali metal is present in a concentration between 1.5% and 4% w/w and aluminosilicate is present in a concentration between 15% and 25%.

3. The composition according to claim 1 or 2, comprising at least one preservative of natural origin, preferably citric acid.

4. The composition according to claim 3, wherein the at least one preservative is present in a concentration between 5 and 12 % w/w, preferably between 7 and 10 % w/w.

5. The composition according to any one of claims 1 to 4, wherein the disaccharide is lactose, the salt of hydrochloric acid is sodium chloride, the salt of carbonic acid is sodium bicarbonate, the at least one amino acid with a hydrophobic side chain is glycine, the alkali metal is potassium and the aluminosilicate is zeolite.

6. The composition according to any one of the preceding claims formulated for oral use, preferably in the form of a powder, or formulated as a liquid, preferably as a syrup or beverage; or it is added to a food.

7. The composition according to any one of claims 1-6 for use as a medicament.

8. The composition according to any one of claims 1-6 for use in the treatment or prevention of a gastrointestinal disorder or condition caused by a gastrointestinal disorder in an individual.

9. The composition for use according to claim 8, wherein the gastrointestinal disorder is selected from: diarrhoea, dysentery, inflammatory bowel disease (IBD), ulcerative colitis, intestinal colopathy, vomiting and combinations thereof.

10. The composition for use according to claim 8, wherein the condition caused by a gastrointestinal disorder is selected from electrolyte dehydration, preferably dehydration following diarrhoea and/or dysentery and metabolic acidosis.

11. The composition for use according to any one of claims 8-10 wherein the individual is an animal, preferably a bovine or a sheep or a pig, more preferably a bovine.

12. A meal replacement comprising the composition according to any one of claims 1-6.
